# EUROPEAN PATENT APPLICATION

(11) **EP 4 079 352 A1**
(43) Date of publication of application: **26.10.2022**
(21) Application number: 21305525.4
(22) Date of filing: 21.04.2021
(51) Int. Cl.: A61M 5/32, A61M 5/50, A61M 5/31

(54) **NEEDLE COVER FOR MEDICAL INJECTION DEVICE**

(71) Applicant: Becton Dickinson France, 38800 Le Pont-de-Claix (FR)
(72) Inventor: ZUCCHELLI, Jeremy, 38400 Saint martin d'hères (FR); RIVIER, Cédric, 38340 VOREPPE (FR)
(74) Representative: Germain Maureau

(57) **Abstract**

A needle cover for protecting a needle mounted on a tip of a medical injection device, in which the tip extends from a distal end of the medical injection device, may include an inner shield extending along a longitudinal axis, and an outer shield surrounding at least partially the inner shield, and operatively connected to said inner shield, in which, when the needle cover is positioned on the tip of the medical injection device, a proximal surface of the inner shield contacts a distal end of the tip of the medical injection device.

## Description

### BACKGROUND OF THE INVENTION

### Field of the Invention

The present disclosure relates generally to medical injection devices for delivery of a fluid or liquid medicament. More particularly, the present disclosure relates to a needle cover adapted to be mounted on a tip of a medical injection device for covering a needle attached thereon. The disclosure also relates to a medical assembly including a medical injection device and a needle cover for enclosing the needle of the medical injection device.

### Description of Related Art

Medical injection devices such as syringes are well known in the art. These devices typically include a container for containing a medical composition such as a liquid medicament. Said container usually includes an end piece in a form of a longitudinal tip defining a fluid path through which the medical solution is expelled. A needle may be attached to the tip in order to prick the patient's skin and to perform the injection of the medical composition.

In order to maintain sterility prior to use and to reduce the risk of incurring an accidental needle-stick, protection of the needle is important. Thus, a needle cover may be mounted on the tip of the barrel so as to enclose the needle. This renders the needle physically inaccessible by the persons around the device. The needle cover may include an inner shield, in a material with elastomeric properties, and may further include an outer shield, in rigid plastic, surrounding the inner shield. In some examples, the inner needle shield may be made of a rubber material. The inner needle shield ensures the sealing of the medical injection device. To that purpose, the inner needle shield includes a sealing portion that sealingly contacts the outer surface of the syringe's tip to provide a tight seal. The inner needle shield prevents any contamination of the medical composition from the outside environment, thereby assuring the container closure integrity. The inner needle shield further prevents any leakage of composition from the outlet of the needle to the external environment. To that purpose, the needle is preferably pricked in the inner needle shield. The rigid outer shield may be made of a rigid or semi-rigid material that surrounds the inner needle shield.

The inner needle shield creates an interference force with the tip of the medical injection device to ensure a full closure of the liquid within the medical injection device. In some examples, the closure integrity of the opening of the medical injection device is created by a radial compression of the inner needle shield on the tip of the medical injection device. The radial compression on the tip of the medical injection device is increased below the tip of the medical injection device using the rigid outer needle shield to avoid any unintentional dislodgement of the needle cover off of the medical injection device. Many of these features of the needle cover also affect the amount of force needed to remove the needle cover from the medical injection device.

When designing a needle cover there are several features to take into consideration that affect the performance level of the needle cover. In particular, among these features are container closure integrity at the tip of the medical injection device, the unintentional dislodgement of the needle cover from the medical injection device, and the ability to pull the needle cover off of the medical injection device using a reasonable amount of force. These features of the needle cover often determine three different characteristics of the needle cover, which often makes it difficult to adjust a performance level for an individual feature without affecting the performance level of the remaining features.

### SUMMARY OF THE INVENTION

In view of the problems identified above, there is a current need for a needle cover that improves the container closure integrity at the tip of the medical injection device, reduces the possibility of an unintentional dislodgement of the needle cover from the medical injection device, and reduces the amount of force needed to remove the needle cover from the medical injection device. There is also a need for a needle cover that enables the tracking of the medical device on which it is mounted from the manufacture or the assembly steps to the final use of said medical device. Indeed, there is a need for providing a device or a needle cover that allows individual identification of a medical injection device. Moreover, there is a need to make sure that a medical injection device contains the expected pharmaceutical composition and may not be filled with another pharmaceutical composition than the original one. There is also a need to ensure that when a medical injection device is up to be used, it has not been opened beforehand. Thus, it may be desirable to provide such a medical injection device with tamper-evidence means.

In one example of the present disclosure, a needle cover for protecting a needle mounted on a tip of a medical injection device, wherein the tip extends from a distal end of the medical injection device, the needle cover, may include: an inner shield extending along a longitudinal axis, and an outer shield surrounding at least partially the inner shield, and operatively connected to said inner shield, the needle cover being characterized in that: when the needle cover is positioned on the tip of the medical injection device, a proximal surface of the inner shield contacts a distal end of the tip of the medical injection device and the proximal surface of the inner shield may create an axial compression in a longitudinal direction on the distal end of the tip of the medical injection device.

In one example of the present disclosure, the proximal surface of the inner shield may contact a distalmost surface of the distal end of the tip of the medical injection device. The inner shield may create an axial compression on the distal end of the tip of the medical injection device and reduces a radial compression on the distal end of the tip of the medical injection device. A proximal-most surface of the inner shield may contact a distalmost surface of the distal end of the tip of the medical injection device. The inner shield may be made of a deformable material. The inner shield may be configured to receive at least a portion of the needle. The outer shield may include at least one hook that assists in removing the inner shield from the tip of the medical injection device when the needle cover is removed from the medical injection device. The at least one hook may include three or four hooks formed on the outer shield. The at least one hook may be made of a flexible material that allows the at least one hook to bend under pressure. The at least one hook may be angled inwardly relative to the longitudinal axis of the inner shield and distally relative to a proximal portion of the tip of the medical injection device. The at least one hook may be configured to contact a shoulder defined on the tip of the medical injection device when the needle cover is positioned on the tip of the medical injection device. The at least one hook may be positioned on a distal end of the outer shield. When the needle cover is removed from the tip of the medical injection device, the at least one hook may be configured to slide along an outer surface of the tip to assist in peeling the inner shield off of the tip.

In one example of the present disclosure, a needle cover for protecting a needle mounted on a tip of a medical injection device, wherein the tip extends from a distal end of the medical injection device, the needle cover, may include: an inner shield extending along a longitudinal axis, and an outer shield surrounding at least partially the inner shield, and operatively connected to said inner shield, the needle cover being characterized in that: the needle cover further comprises a tamper evidence ring that identifies whether the needle cover has been removed from the tip of the medical injection device.

In one example of the present disclosure, the tamper evidence ring may include at least one of the following: a Bluetooth tag, a radio frequency identification (RFID) tag, an ultra-wide-band real-time location system (RTLS), a Wi-Fi RTLS, and an infrared RTLS. The RFID tag may be overmolded with the tamper evidence ring. The tamper evidence ring may include a lower portion and an upper portion, wherein each of the lower portion and the upper portion include a portion of an antenna. A conductive bridge may be established between the antenna portions of the lower portion and the upper portion of the tamper evidence ring. The tamper evidence ring may include a bridge that establishes a breakable connection between the tamper evidence ring and the medical injection device.

In one example of the present disclosure, a medical injection device may include a barrel extending a proximal face to a distal face, wherein a tip is provided on the distal face of the barrel; and a needle cover for protecting a needle mounted on the tip of the medical injection device, the needle cover, comprising an inner shield extending along a longitudinal axis, and an outer shield surrounding at least partially the inner shield, and operatively connected to said inner shield, the medical injection device being characterized in that when the needle cover is positioned on the tip of the medical injection device, a proximal surface of the inner shield contacts a distal end of the tip of the medical injection device, and the proximal surface of the inner shield creates an axial compression on the distal end of the tip of the medical injection device.

In one example of the present disclosure, a medical injection device may include a barrel extending a proximal face to a distal face, wherein a tip is provided on the distal face of the barrel; and a needle cover for protecting a needle mounted on the tip of the medical injection device, the needle cover, comprising an inner shield extending along a longitudinal axis, and an outer shield surrounding at least partially the inner shield, and operatively connected to said inner shield, the medical injection device being characterized in that the outer shield includes at least one hook that assists in removing the inner shield from the tip of the medical injection device when the needle cover is removed from the medical injection device.
In one example of the present disclosure, a medical injection device may include a barrel extending a proximal face to a distal face, wherein a tip is provided on the distal face of the barrel; and a needle cover for protecting a needle mounted on the tip of the medical injection device, the needle cover, comprising an inner shield extending along a longitudinal axis, and an outer shield surrounding at least partially the inner shield, and operatively connected to said inner shield, the medical injection device being characterized in that the needle cover further comprises a tamper evidence ring that identifies whether the needle cover has been removed from the tip of the medical injection device.

### BRIEF DESCRIPTION OF THE DRAWINGS

FIG. 1 is a perspective view of a medical injection device according to one example of the present disclosure;
FIG. 2 is a cross-sectional view of the medical injection device of FIG. 1;
FIG. 3 is another cross-sectional view of the medical injection device of FIG. 1;
FIG. 4 is a cross-sectional view of a needle cover according to one example of the present disclosure;
FIG. 5 is a perspective view of a medical injection device according to one example of the present disclosure;
FIG. 6 is a side view of the needle cover and tamper evidence ring of FIG. 5 in which the tamper evidence ring is unbroken;
FIG. 7 is a side view of the needle cover and tamper evidence ring of FIG. 5 in which the tamper evidence ring is broken;
FIG. 8 is a cross-sectional view of FIG. 6 in which the tamper evidence ring is unbroken; and
FIG. 9 is a cross-sectional view of FIG. 7 in which the tamper evidence ring is broken.

### DESCRIPTION OF THE DISCLOSURE

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the following discussion, "distal" refers to a direction generally toward an end of a medical injection device adapted for contact with a patient's skin, and "proximal" refers to the opposite direction of distal, i.e., away from the end of a medical injection device. In other words, the "distal direction" is to be understood as meaning the direction of injection. The distal direction corresponds to the travel direction of the plunger during the injection, the medical composition contained initially in the barrel being expelled from the latter. The "proximal direction" is to be understood as meaning the opposite direction to said direction of injection. For purposes of this disclosure, the above-mentioned references are used in the description of the components of a medical injection device in accordance with the present disclosure.

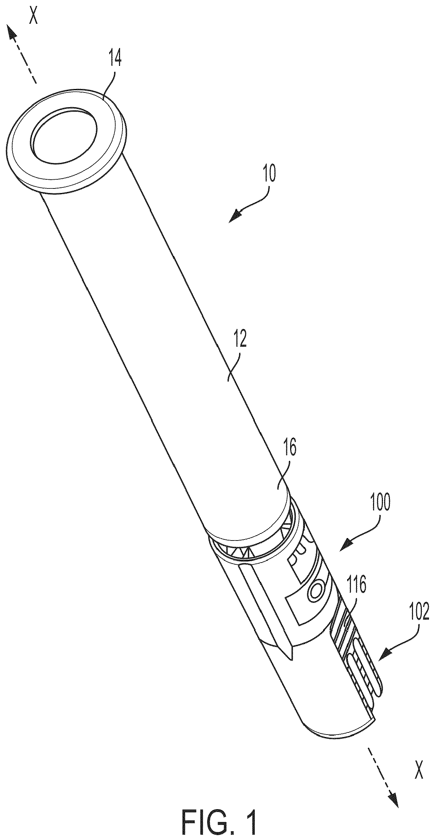

Referring to FIGS. 1-3, the medical injection device 10 may include a barrel 12 extending along a longitudinal axis X from a proximal face 14 to a distal face 16, defining a reservoir 18 for a medical composition such as a liquid medicament. The medical injection device 10 may also include a stopper (not shown), and a plunger rod (not shown) translationally movable inside the barrel 12 from a proximal position to a distal position for injecting the composition.

The medical injection device 10 further includes a distal tip 20 extending along the longitudinal axis X from the distal face 16 of the barrel 12. The distal tip 20 may be at least partially hollow so as to form a fluid path in fluidic communication with the barrel 12. A needle 22 may be attached to the distal tip 20 of the medical injection device 10 and may be in fluid communication with the fluid path. It is noted herein that the distal face 16 of the barrel 12 is proximate a shoulder of the medical injection device 10.

The medical injection device 10 is preferably made of glass, and more preferably is a glass syringe. Such glass syringes are largely used in hospital environments and readily sterilizable. In other examples of the present disclosure, the medical injection device 10 may be made of a medical grade plastic. The medical injection device 10 is preferably a prefilled or a pre-fillable syringe. The medical injection device 10 is more preferably a syringe with the needle 22.

With continued reference to FIGS. 1-3, the medical injection device 10 may further include a needle cover 100. The needle cover 100 may shield and cover the needle 22. The needle cover 100 may include an outer shield 102 and an inner shield 104 that covers the needle 22. In one embodiment, the outer shield 102 covers at least a portion of the inner shield 104. In another embodiment, the outer shield 102 covers the entire inner shield 104.

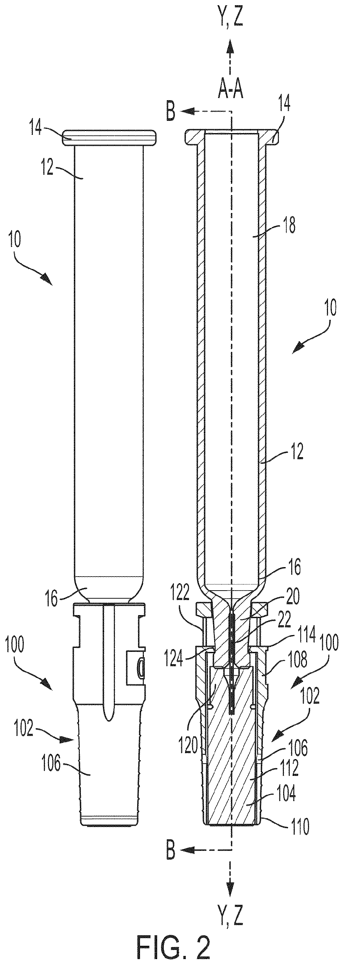

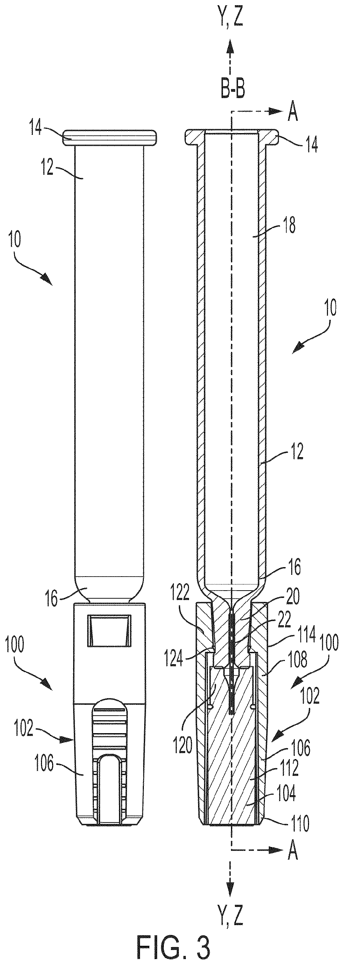

Referring to FIGS. 2 and 3, the outer shield 102 in accordance with an example of the present disclosure is described in greater detail. The outer shield 102 is preferably made in a rigid material, such as rigid plastic. The outer shield 102 may be made of a medical grade plastic. The rigid material confers rigidity to the outer shield 102, which allows said outer shield 102 to better protect the needle cover 100 from shocks. The structural integrity of the needle cover 100 is thereby improved.

The outer shield 102 includes a body 106 having a proximal end 108 and a distal end 110. The body 106 extends along a longitudinal axis Y. The longitudinal axis Y coincides with the longitudinal axis X when the needle cover 100 is mounted on the tip 20 of the medical injection device 10.

In one example, the body 106 may be substantially cylindrical in shape. The body 106 may define an inner cavity 112 that at least partially receives the inner shield 104 of the needle cover 100. A collar 114 may be formed on the proximal end 108 of the outer shield 102. The collar 114 may have a larger diameter than the remaining portion of the body 106. In one example, a plurality of ribs and grooves 116 may be defined on the body 106 of the outer shield 102. The ribs and grooves 116 may be provided adjacent the distal end 110 of the outer shield 102. The ribs and grooves 116 provide a gripping surface for a user to use when pulling the outer shield 102 away from the medical injection device 10. In one embodiment, the distal end 110 of the outer shield 102 may include at least one opening for providing sterilization to the needle cover 100.

With continued reference to FIGS. 2 and 3, the inner shield 104 according to one example of the present disclosure is described. The inner shield 104 may extend along a longitudinal axis Z. The longitudinal axis Z may coincide with the longitudinal axis Y when the inner shield 104 is at least partly enclosed in the outer shield 102. The inner shield 104 preferably has a cylindrical shape and a circular cross section.

The inner shield 104 is preferably made in a material with elastomeric properties. In this way, at least a portion of the inner shield 104 may slightly deform when connecting the inner shield 104 to the medical injection device 10 so as to match the shape of the tip 20. Meanwhile, the needle tip 20, or distal part of the needle 22, may penetrate the inner shield 104. This further reduces the risk of leakage of the medical composition via the needle 22 to the external environment. The material with elastomeric properties is preferably a thermoplastic elastomer, an elastomer, or a rubber. Preferably, the material with elastomeric properties is sterilizable.

In one embodiment, the outer shield 102 surrounds at least partially the inner shield 104. The outer shield 102 is fixed to the inner shield 104. The inner shield 104 and the outer shield 102 may be fixed together by a snap-fit connection or a friction fit, among other connection methods. When the needle cover 100 is mounted on the medical injection device 10, the inner shield 104 encloses at least a portion of the tip 20 of the barrel 12. The needle cover 100 is thus tightly and sealingly connected to the tip 20. In one embodiment, the inner shield 104 creates a sufficiently tight seal with the tip 20 of the medical injection device 10 to ensure the sterility and fluid tight seal between the tip 20 of the medical injection device 10 and the inner shield 104.

In order to mount the needle cover 100 on the medical injection device 10, the outer shield 102 is first placed over the inner shield 104, and then the entire needle cover 100 is mounted on the medical injection device 10. After the needle cover 100 has been positioned on the medical injection device 10, the needle cover 100 can be moved in a distal direction to remove it from the medical injection device 10. As a pulling force is applied to the outer shield 102 by a user, the outer shield 102 is moved in the distal direction along with the inner shield 104. As the outer shield 102 is moved in the distal direction, a proximal end 108 of outer shield 102 will begin to contact and abut a proximal portion 120 of the inner shield 104. Once the proximal end 108 of the outer shield 102 contacts the proximal portion 120 of the inner shield 104, the proximal end 108 begins to push the proximal portion 120 in a distal direction. As the proximal portion 120 is pushed in the distal direction, the inner shield 104 is pulled in a radial direction away from the medical injection device 10.

With continued reference to FIGS. 2 and 3, according to one example of the present disclosure, the inner shield 104 may be positioned to reduce a radial compression force that is applied to the tip 20 of the medical injection device 10. In one example of the present disclosure, instead of the inner shield 104 gripping a side surface of the tip 20 of the medical injection device 10, the inner shield 104 is positioned to engage the distalmost surface of the tip 20 of the medical injection device 10. In this arrangement, once the needle cover 100 has been positioned on the medical injection device 10, the proximal end of the inner shield 104 engages the distalmost surface of the tip 20 of the medical injection device 10 to create a liquid tight seal to prevent fluid from leaking from the medical injection device 10. In one example of the present disclosure, the inner shield 104 creates an axial compression in a longitudinal direction on the tip 20 of the medical injection device 10. The inner shield 104 may deform under the axial compression to create the seal. In one example of the present disclosure, a shoulder that extends radially from the tip 20 may also be formed to receive the inner shield 104 to create an axial compression on the tip 20.

By creating the seal with the distalmost surface of the tip 20 of the medical injection device 10, the inner shield 104 does not engage with a side surface of the tip 20 and does not create variations in the amount of force needed to pull the needle cover 100 off of the medical injection device 10. When the inner shield 104 grips a side surface of the tip 20, the amount of force needed to pull the inner shield 104 away from the tip 20 may vary based on the radial compression force vectors that are created on the side surface of the tip 20. Therefore, by reducing or eliminating the radial compression forces that are created by the inner shield 104 gripping the side surface of the tip 20, the amount of force needed to pull the needle cover 100 off of the tip 20 is more consistent and predictable. Instead, the amount of force needed to pull the needle cover 100 off of the medical injection device 10 is more consistently maintained by creating a positive engagement between the proximal end 108 of the outer shield 102 and the tip 20 of the medical injection device 10. In one example of the present disclosure, the proximal end 108 of the outer shield 102 defines at least two openings 122. When positioning the needle cover 100 on the tip 20, a shoulder 124 formed on the tip 20 may engage a wall of the openings 122 to create a positive locking engagement. In order to remove the needle cover 100 from the tip 20, a sufficient pull-out force to pull the needle cover 100 from the tip 20 is needed to pull the openings 122 past the shoulder 124 on the tip 20.

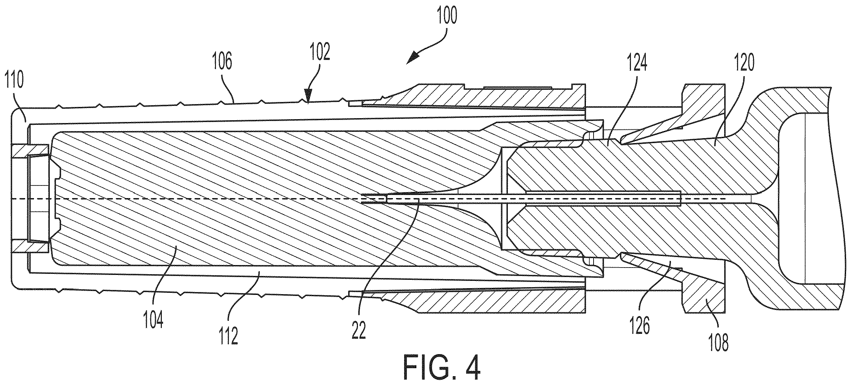

With reference to FIG. 4, in one example of the present disclosure, the needle cover 100 may also include at least one hook 126 on the rigid outer shield 102 to assist in more consistently removing the inner shield 104 from the tip 20 of the medical injection device 10. In one example of the present disclosure, the rigid outer shield 102 may include two hooks 126. In one example of the present disclosure, the rigid outer shield 102 may include four hooks 126. It is also contemplated that additional or fewer hooks 126 may be provided on the rigid outer shield 102. The hooks 126 may be angled inwardly towards the tip 20 of the medical injection device 10 relative to the longitudinal axis X of the medical injection device 10. In one example of the present disclosure, the hooks 126 may be made of a material that permits the hooks 126 to bend outwardly when the needle cover 100 is slid onto the tip 20. In one embodiment, the hooks 126 may be made as the same material of the outer shield 102. The shoulder 124 of the tip 20 may force the hooks 126 outwardly when the needle cover 100 is slid onto the tip 20. Once the hooks 126 have been moved past the shoulder 124, the hooks 126 may spring inwardly again to engage an undercut on the shoulder 124 to prevent removal of the needle cover 100 from the tip 20. In order to remove the needle cover 100 from the tip 20, a sufficient amount of force to pull the needle cover 100 from the tip 20 is needed to pull the hooks 126 past the shoulder 124 on the tip 20. The hooks 126 also assist in peeling the inner shield 104 away from the tip 20 when removing the needle cover 100. As the hooks 126 move in a distal direction away from the tip 20, the hooks 126 are directed along an outer surface of the tip 20 in a distal direction. The hooks 126 will make contact with the inner shield 104 that is held on the tip 20 and will begin to peel the inner shield 104 away from the tip 20 to reduce the amount of force needed to pull the inner shield 104 away from the tip 20 of the medical injection device 10.

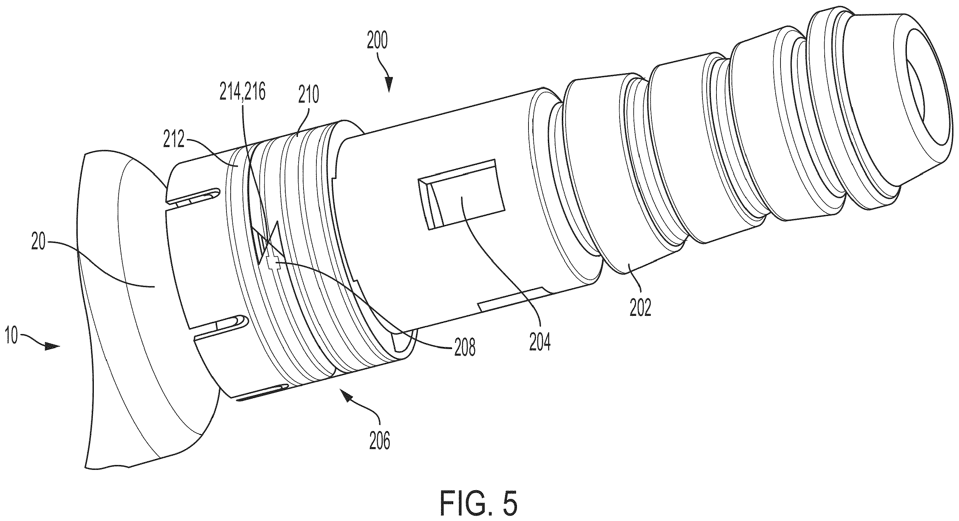

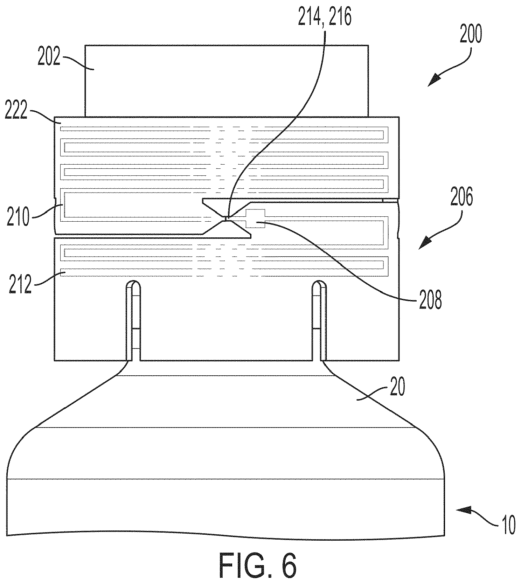

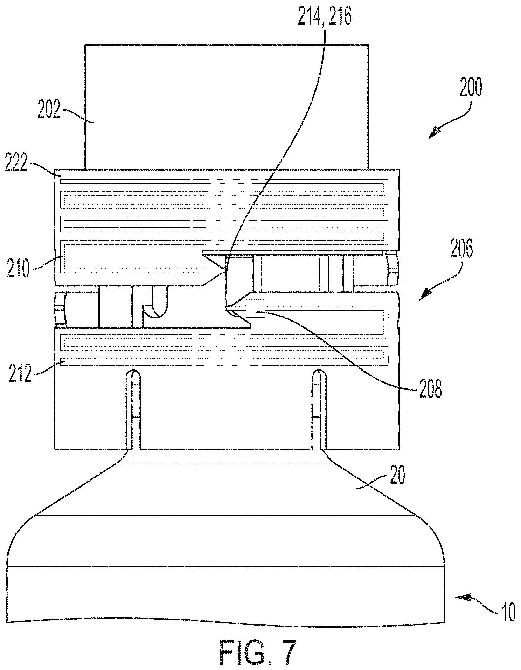

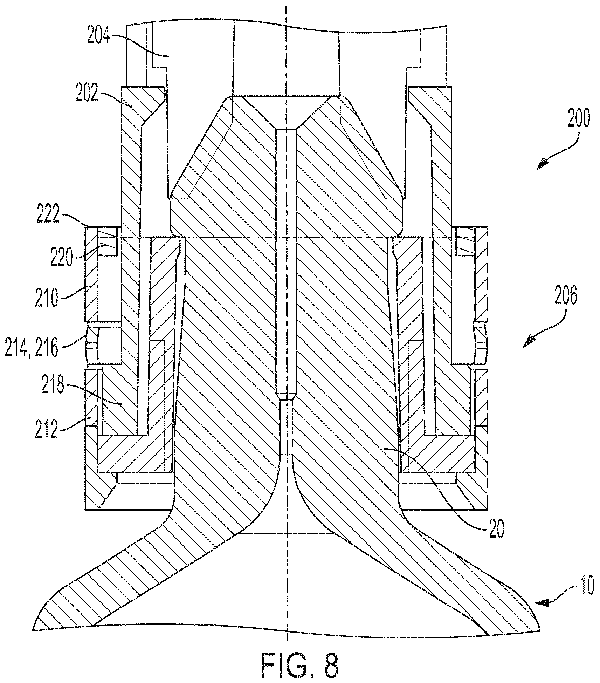

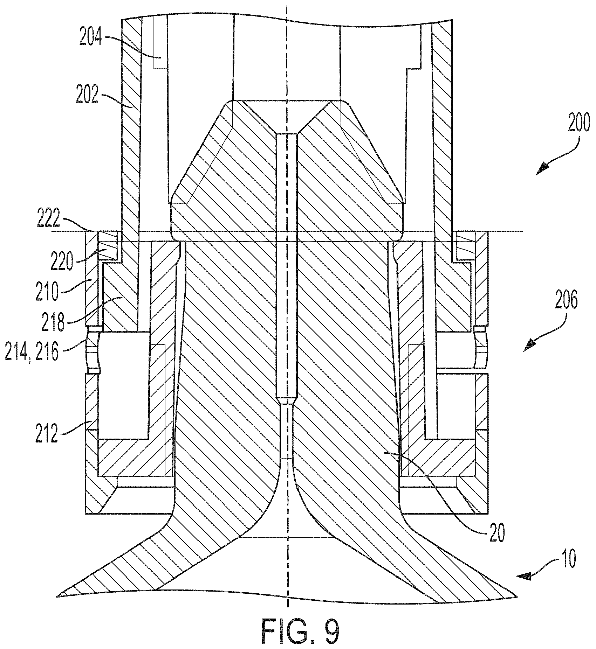

With reference to FIGS. 5-9, in one example of the present disclosure, a needle cover 200 may also include tamper evidence and device identifier features. In one example of the present disclosure, in the event the needle cover 200 has been removed from the tip 20 of the medical injection device 10, an indication of the current status of the medical injection device 10, whether the needle cover 200 has been removed therefrom, may be provided. The indication of the status of the medical injection device 10 may be a visual indicator, an auditory indicator, or any other method of indicating a current status to an individual. As long as the needle cover 200 has not been removed from the tip 20 of the medical injection device 10, the needle cover 200 will include a detectable unique device identifier that enables tracking of the needle cover 200 between the manufacturing of the medical injection device 10 at the manufacturer's facility until the medical injection device 10 is delivered to a secondary packaging facility and then to its final destination, such as a pharmaceutical lab, a hospital, a treatment center, or any other location at which the medical injection device 10 may be used.

In one example of the present disclosure, the needle cover 200 may include a rigid outer shield 202, a deformable inner shield 204, and a tamper evidence ring 206 provided thereon. The tamper evidence ring 206 may include an RFID (radio frequency identification) tag 208. In some examples of the present disclosure, the RFID tag 208 may be an ultrahigh frequency (UHF) RFID tag. In some embodiments, the tamper evidence ring 206 may include an ultra-wide-band real-time location system (RTLS), a Wi-Fi RTLS, and/or an infrared RTLS. In some examples, the RFID tag may be in the form of a wet inlay, dry inlay, or pressure sensitive label. RFID wet inlays and RFID dry inlays can include a substrate, for example made of paper or polyethylene terephthalate (PET). The RFID tag can be disposed on one side of the substrate. RFID wet inlays and RFID dry inlays can further includes at least one protective layer on top of the RFID tag. The protective layer can be a siliconized paper. RFID wet inlays are described as "wet" as they include an adhesive layer on the other side of the substrate and a backing paper, for example with a silicon liner. RFID dry inlays are described as "Dry" due to their lack of adhesive backing. Pressure-sensitive labels are analogous to a high-tech sticker. In one embodiment, the RFID tag is a High Frequency Radio Frequency Identification (HF-RFID) tag. High frequencies are usually about 1-15 MHz. In this embodiment, a RFID reader can for example read the HF-RFID tag at a distance about one meter. Preferably, the RFID tag is an Ultra High Frequency Radio Frequency Identification (UHF-RFID) tag. Ultra-high frequencies are usually about 400-1000MHz. In this embodiment, a RFID reader can for example read the UHF-RFID tag at a distance about fifteen meters.

Preferably, the RFID antenna has at least one leg. The RFID antenna may have two or four legs for example. More preferably, the RFID antenna has two legs, each leg having an extremity. For example, the RFID antenna has two legs which may have a plurality of steady steps. According to the present invention, steady step means that the two legs of the RFID antenna may have a plurality of even steps. For example, the steady step forms a square, rectangle, triangle or wave shape. Preferably, the plurality of steady steps have the same amplitude. For example, the RFID antenna has two sinusoidal shaped legs, straight-shaped legs or coil-shaped legs. Preferably, when the RFID antenna has two sinusoidal shaped legs, both legs of the RFID antenna being made of a plurality of sinusoids. For example, when the RFID tag is an UHF-RFID tag, the two legs have sinusoidal shape. For example, when the RFID tag is a HF-RFID tag, the two legs have a coil shape. Indeed, it is believed that in these embodiments, the shape of the legs further improves the communication between the chip, the antenna of the RFID tag and the RFID reader.

Preferably, the RFID antenna forms a loop between the legs, the RFID chip being located between the loop and the legs of the RFID antenna. In one embodiment, the RFID tag is dissymmetrical, i.e. the RFID chip is not located at equidistance of both RFID antenna extremities. Preferably, the RFID chip is located at equidistance of both RFID antenna extremities.

In some examples of the present disclosure, the tamper evidence ring 206 may be made of polyethylene terephthalate or any other material that permits material to adhere to the tamper evidence ring 206. The tamper evidence ring 206 may include, in a lower portion for example, the RFID tag 208 and a first part of an antenna 210. In an upper portion of the tamper evidence ring 206, for example, a second part of the antenna 212 may be positioned in the tamper evidence ring 206. In an intermediate portion of the tamper evidence ring 206, for example, an electric bridge 214 may be positioned to connect the first part of the antenna 210 to the second part of the antenna 212. In one example of the present disclosure, the RFID tag 208 and antennas 210, 212 may be over-molded or glued into the tamper evidence ring 206 during manufacturing of the tamper evidence ring 206. In some examples of the present disclosure, the RFID tag 208 may be maintained by a vacuum in the tamper evidence ring 206, instead of an electrostatic mechanism, so as to avoid damage to the RFID tag 208. To improve the readability of the RFID tag 208 from multiple angles, the RFID tag 208 may extend along at least half of the surface of the tamper evidence ring 206, such as 180 degrees around the circumference of the tamper evidence ring 206.

In one example of the present disclosure, the tamper evidence ring 206 may include a storage space to write a unique device identification (UDI) tag for the needle cover 200. The storage space may be password protected so only authorized individuals may set the UDI tag. In one example of the present disclosure, the upper part of the tamper evidence ring 206 may be linked to the lower part of the tamper evidence ring 206 via a bridge 216 that extends circumferentially around the tamper evidence ring 206. Upon a sufficient force being applied to the tamper evidence ring 206, the bridge 216 is configured to break to separate the upper part of the tamper evidence ring 206 from the lower part of the tamper evidence ring 206. In one example of the present disclosure, the lower part of the tamper evidence ring 206 may be operatively connected to the medical injection device 10, while the upper part of the tamper evidence ring 206 may be operatively connected to the needle cover 200. The tamper evidence ring 206 portions may be operatively connected to the medical injection device 10 and the needle cover 200 via welding, an adhesive, clips, or any other connection method that secures the tamper evidence ring 206 to these devices. By breaking the bridge 216 under pressure, the tamper evidence ring 206 is configured to indicate that the needle cover 200 has been dislodged from the medical injection device 10. As the needle cover 200 is pulled off of the medical injection device 10, the bridge 216 is configured to break, thereby disconnecting the antennas 210, 212 and deactivating the RFID tag 208. Therefore, an indication that the needle cover 200 has been removed from the medical injection device 10 may be notified to an individual, or a user wanting to detect the medical injection device 10 would not be able to detect it any longer. A proximal end 218 of the outer shield 202 then comes into contact with a shoulder 220 extending inwardly from a distal end 222 of the tamper evidence ring 206 to pull the upper portion 210 of the tamper evidence ring 206 away from the tip 20 of the medical injection device 10 with the needle cover 200. A traction force is established between the proximal end 218 of the outer shield 202 and the shoulder 220 of the tamper evidence ring 206 to remove the tamper evidence ring 206 along with the needle cover 200.

In one example of the present disclosure, before removing the needle cover 200 from the medical injection device 10 for the first time, the RFID tag 208 remains intact and may be read by an RFID reader to identify unique device identification between the manufacturing of the needle cover 200 and the removal of the needle cover 200 from the medical injection device 10. After the needle cover 200 has been removed from the medical injection device 10, the RFID tag 208 will no longer transmit a signal. Therefore, when an individual uses an RFID reader and picks up a signal from the RFID tag 208, this is an indication to the individual that the needle cover 200 has not yet been removed from the medical injection device 10. When the individual uses an RFID reader and does not pick up a signal from the RFID tag 208, this is an indication to the individual that the needle cover 200 has been removed from the medical injection device 10. Using the RFID reader, the individual can quickly determine for multiple medical injection devices 10 whether the needle covers 200 have been removed therefrom. This configuration also permits the individual to ensure the needle covers 200 are intact on the medical injection devices 10 without opening the product packaging. No line-of-sight is required to determine this connection between the needle cover 200 and the medical injection device 10.

All of the components of the medical injection device 10 may be constructed of any known material, and are desirably constructed of medical-grade polymers.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A needle cover for protecting a needle mounted on a tip of a medical injection device, wherein the tip extends from a distal end of the medical injection device, the needle cover, comprising:
an inner shield extending along a longitudinal axis, and
an outer shield surrounding at least partially the inner shield, and operatively connected to said inner shield,
the needle cover being **characterized in that**:
when the needle cover is positioned on the tip of the medical injection device, a proximal surface of the inner shield contacts a distal end of the tip of the medical injection device, and
the proximal surface of the inner shield creates an axial compression in a longitudinal direction on the distal end of the tip of the medical injection device.

2. The needle cover of claim 1, wherein the proximal surface of the inner shield contacts a distalmost surface of the distal end of the tip of the medical injection device.

3. The needle cover of claim 1 or claim 2, wherein the inner shield creates an axial compression on the distal end of the tip of the medical injection device and reduces a radial compression on the distal end of the tip of the medical injection device.

4. The needle cover of any of claims 1-3, wherein a proximal-most surface of the inner shield contacts a distalmost surface of the distal end of the tip of the medical injection device.

5. The needle cover of any of claims 1-4, wherein the inner shield is made of a deformable material.

6. The needle cover of any of claims 1-5, wherein the inner shield is configured to receive at least a portion of the needle.

7. The needle cover of any of claims 1-6, wherein the outer shield includes at least one hook that assists in removing the inner shield from the tip of the medical injection device when the needle cover is removed from the medical injection device.

8. The needle cover of claim 7, wherein the at least one hook comprises three or four hooks formed on the outer shield.

9. The needle cover of claim 7 or claim 8, wherein the at least one hook is made of a flexible material that allows the at least one hook to bend under pressure.

10. The needle cover of any of claims 7-9, wherein the at least one hook is angled inwardly relative to the longitudinal axis of the inner shield and distally relative to a proximal portion of the tip of the medical injection device.

11. The needle cover of any of claims 7-10, wherein the at least one hook is configured to contact a shoulder defined on the tip of the medical injection device when the needle cover is positioned on the tip of the medical injection device.

12. The needle cover of any of claims 7-11, wherein the at least one hook is positioned on a distal end of the outer shield.

13. The needle cover of any of claims 7-12, wherein, when the needle cover is removed from the tip of the medical injection device, the at least one hook is configured to slide along an outer surface of the tip to assist in peeling the inner shield off of the tip.

14. A needle cover for protecting a needle mounted on a tip of a medical injection device, wherein the tip extends from a distal end of the medical injection device, the needle cover, comprising:
an inner shield extending along a longitudinal axis, and
an outer shield surrounding at least partially the inner shield, and operatively connected to said inner shield,
the needle cover being **characterized in that**:
the needle cover further comprises a tamper evidence ring that identifies whether the needle cover has been removed from the tip of the medical injection device.

15. The needle cover of claim 14, wherein the tamper evidence ring comprises at least one of the following: a Bluetooth tag, a radio frequency identification (RFID) tag, an ultra-wide-band real-time location system (RTLS), a Wi-Fi RTLS, and an infrared RTLS.

16. The needle cover of claim 15, wherein the RFID tag is overmolded with the tamper evidence ring.

17. The needle cover of any of claims 14-16, wherein the tamper evidence ring comprises a lower portion and an upper portion, wherein each of the lower portion and the upper portion include a portion of an antenna.

18. The needle cover of claim 17, wherein a conductive bridge is established between the antenna portions of the lower portion and the upper portion of the tamper evidence ring.

19. The needle cover of any of claims 14-18, wherein the tamper evidence ring further comprises a bridge that establishes a breakable connection between the tamper evidence ring and the medical injection device.

20. A medical injection device, comprising:
a barrel extending a proximal face to a distal face, wherein a tip is provided on the distal face of the barrel; and
a needle cover for protecting a needle mounted on the tip of the medical injection device, the needle cover, comprising:
an inner shield extending along a longitudinal axis, and
an outer shield surrounding at least partially the inner shield, and operatively connected to said inner shield,
the medical injection device being **characterized in that**:
when the needle cover is positioned on the tip of the medical injection device, a proximal surface of the inner shield contacts a distal end of the tip of the medical injection device, and
the proximal surface of the inner shield creates an axial compression on the distal end of the tip of the medical injection device.

21. A medical injection device, comprising:
a barrel extending a proximal face to a distal face, wherein a tip is provided on the distal face of the barrel; and
a needle cover for protecting a needle mounted on the tip of the medical injection device, the needle cover, comprising:
an inner shield extending along a longitudinal axis, and
an outer shield surrounding at least partially the inner shield, and operatively connected to said inner shield,
the medical injection device being **characterized in that**:
the outer shield includes at least one hook that assists in removing the inner shield from the tip of the medical injection device when the needle cover is removed from the medical injection device.

22. A medical injection device, comprising:
a barrel extending a proximal face to a distal face, wherein a tip is provided on the distal face of the barrel; and
a needle cover for protecting a needle mounted on the tip of the medical injection device, the needle cover, comprising:
an inner shield extending along a longitudinal axis, and
an outer shield surrounding at least partially the inner shield, and operatively connected to said inner shield,
the medical injection device being **characterized in that**:
the needle cover further comprises a tamper evidence ring that identifies whether the needle cover has been removed from the tip of the medical injection device.
